Europäisches Patentamt

European Patent Office

Office européen des brevets

(11) Publication number: **0 472 003 A1**

# EUROPEAN PATENT APPLICATION

(21) Application number: **91112584.7**

(22) Date of filing: **26.07.91**

(51) Int. Cl.5: **A61K 35/54**, A61K 33/18

(30) Priority: **20.08.90 JP 218580/90**

(43) Date of publication of application:
**26.02.92 Bulletin 92/09**

(84) Designated Contracting States:
**AT BE CH DE DK FR GB IT LI LU NL SE**

(71) Applicant: **NIHON NOSAN KOGYO K.K.**
**No. 11-20, Kitasaiwai 1-chome, Nishi-ku**
**Yokohama-shi, Kanagawa-ken(JP)**

(72) Inventor: **Kunitoshi, Sekimoto**
**No. 1788-9, Kiso-cho**
**Machida-shi, Tokyo-to(JP)**
Inventor: **Tadashi, Ishikawa**
**No. 14-6, Asahi-cho**
**Sagamihara-shi, Kanagawa-ken(JP)**

(74) Representative: **Liedl, Gerhard, Dipl.-Phys.**
**Patentanwalt Herterichstrasse 18**
**W-8000 München 71(DE)**

(54) Anti-inflammatory composite containing iodine enriched eggs.

(57) Use of iodine-enriched eggs in the manufacture of a medicine for treatment of inflammatory diseases is disclosed. The medicine composed of iodine-enriched eggs is free from any adverse reaction and can be ingested as a diet every day for enhancing the adrenal function and promoting the secretion of adrenal steroid hormone, thus being effective for treatment and prevention of inflammatory diseases. It has not only anti-allergic effect but also anti-inflammatory effect against any diseases due to acid metabolism product of fatty acid, which include allergic inflammation diseases.

EP 0 472 003 A1

This invention relates to an anti-inflammatory composite using an iodine-enriched egg as an effective component, that is to say, it relates to a new use of an iodine-enriched egg in manufacturing a medicine for the treatment of inflammatory diseases.

The term "inflammation" generally referres to peccant phenomena topically induced in a living body on account of various stimuli, and there are two inflammatory cases. In one of the two cases, inflammation is brought about by external stimulus such as pollen, ticks and exhaust gas, which acts as allergen and causes allergy via chemical mediators such as histamine and serotonin. In the other of two cases, inflammation is brought about by internal stimulus such as prostaglandin or leucotriene metabolically produced from arachidonic acid, that is to say, an unsaturated fatty acid contained in food, which causes an allergic reaction.

As anti-inflammatory agents against these inflammatory cases, there are those of non-steriod type and those of adrenal steroid type. As an agent of the non-steroid type, there are aspirin, indomethacin, phenyl acetate compounds and the like. As an agent of the adrenal steroid type, there are hydrocortisone, dexamethasone and so on.

These anti-inflammatory agents are effective for such as disease control disturbance in circulatory system, bronchial asthma, hepatitis, nephrotic syndrome, rheumatism, gout, multiple myositis, atopic dermatitis, urticaria,lymphoma, allergic rhinitis and so forth.

The agents noted above, however, have well-known side or adverse effects or reactions. It is well known that the agents of non-steroid type cause gastrointestinal impairment, and that those of the adrenal steroid type cause deterioration of the adrenal function. Accordingly, development of a curative, which is effective for the said diseases and is free from any adverse reaction, has been desired.

In the light of the above situation, the inventors have conducted extensive researches and investigations and found that giving iodine-enriched egg for a predetermined period of time is effective not only against allergic diseases due to allergens as external stimulus but also against other inflammatory diseases through the medium of prostaglandin, leucotriene and the like as internal stimulus, and the present invention is based on this finding.

An object of the invention is to provide a composite, which is free from any adverse reaction, and daily ingestion of which as a diet has an effect of enhancing the adrenal function so as to promote secretion of the adrenal steroid hormone, thus being effective not only for treatment of allergic inflammation diseases such as allergic rhinitis, gout and rheumatism but also for treatment of other inflammatory diseases due to metabolical production of unsaturated fatty acid.

To attain the above object of the invention, there is provided a composite having an anti-inflammatory effect, which contains iodine-enriched egg as an effective component.

According to searches by the inventors, there is no report that iodine and iodine-enriched egg have an anti-inflammatory effect, and the present invention is a precursory invention in this sense.

Figure 1 is a graph showing change of histamine amount in overall blood of allergic asthma rats which were under an antigen absorption challenging test with dosing the composite according to the invention;

Figure 2 is a graph showing changes of histamine amount in lung of the rats in the same challenging test;

Figure 3 is a graph showing reduction of lysosomal enzyme activity in nosal cavity perfusate of allergic rhinitis rats which were under a nosal perfusing test with dosing the composite according to the invention;

Figure 4 is a graph showing changes in lysosomal enzyme activity in perfusate in the same perfusing test;

Figure 5 is a graph showing sneeze suppression effect in allergic rhinitis patients who ingested the composite according to the invention;

Figure 6 is a graph showing nasal obstruction suppression effect in the same patients; and

Figure 7 is a graph showing reduction of lysosomal enzyme activity in pituita of the same patients.

The iodine-enriched egg as an effective component of the composite according to the invention usually can be obtained as follows. The feed for domestic fowl is mixed with no less than a predetermined amount of iodine compounds such as calcium iodate, potassium iodate, potassium iodide, sodium iodide and the like, and/or marine algae containing much iodine such as wakame seaweed, kelp, hijiki seaweed and the like, either without any process or after necessary process. The egg-laying fowl is fed with the feed, so that the iodine content in the egg is increased. Among the iodine compounds, , calcium iodate is most preferred. Further preferably, it may be used in combination with marine algae such as tangleweed and the like. Although the amount of iodine given to fowl varies in dependence on the kind of fowl, such as hen and quail, usually the feed may contain 20 to 2,500 ppm, preferably 20 to 100 ppm of iodine.

By feeding fowls with the above feed containing much iodine or iodine compounds, the iodine-enriched egg (stated often as IEE hereinafter) can be produced.

In case of laying hen, for example, by providing the feed containing about 20 ppm of iodine, it is possible to obtain iodine-enriched eggs containing about 100 $\mu$g of iodine per egg after a week. Also, by giving the feed containing about 100 ppm of iodine, iodine-enriched eggs can be produced, which contain about 600 to 1000 $\mu$g of iodine per egg.

The iodine-enriched egg obtained in the above way can be used as the composite of the invention under non-processed form. However, it may be used after necessary process such as drying, condensation and powdering. Furthere, it may be used in the form of tablets, powder, granules, etc. by mixing with suitable shape-forming agents and/or binders.

The iodine-enriched egg as an effective component of the composite of the invention presents no safety problem, because the living body of fowl provides vivifiltration. As a usual diet one or two eggs per day can be ingested without any dangerous effect on health.

As the composite according to the invention does not present quick effectiveness but slow effectiveness, it is preferably ingested continuously for one week or more by animals, and three weeks or more by humans.

By continuously ingesting the composite of the invention in an amount of 100 to 500 $\mu$g as iodine in egg, an adult can be protected from various inflammatory diseases or the diseases can be considerably alleviated.

For instance, in case of allergic diseases, the function and effect of the composite according to the invention can be confirmed by spraying $\alpha$-amylase to rats for absorption as allergen and then determing the amount of histamine discharged from nasal mucous membrane or lung fat tissues, or confirmed by determing the value of lysosomal enzyme activity in pituita in an sensitivity experiment on allergic rhinitis rats sensitizied with anti-bovine serum albumin serum as allergen.

In case where prostaglandin or leucotriene causes inflammation and thereby allergic reaction is brought about, the said function and effect can be confirmed by coating arachidonic acid on the external ear of mouse so as to be absorbed and metabolized through the skin, and then checking whether the ear is suppressed from getting fat and thick, and also by measuring the adrenal steroid hormone value in blood.

As has been described in the foregoing, because the composite of the invention contains iodine-enriched egg as an effective component, it is free from any adverse reaction and can be a diet to be ingested every day for activating and enhancing the adrenal function and thereby promoting the secretion of adrenal steroid hormone, and thus being effective for treatment and prevention of inflammatory diseases. Further, it has not only an anti-allergic effect but also anti-inflammatory effect against any diseases due to metabolism product of fatty acid, which includes allergic inflammation diseases.

Animal Experiment

Animal experiments concering anti-inflammatory effect of the composite according to the invention were conducted. As a model of animal experiment, inflammation challenging method by arachidonic acid was used.

In this method, albumin-coupling pigment is produced in advance in the body of mouse by doing Evans blue treatment. Then, arachidonic acid is coated on the external ear of mouse. Arachidonic acid is absorbed through the skin of ear, and then leucotriene (LT) and prostaglandin (PG), these being strong inflammation causing substances, are synthesized in local places through arachidonic acid metabolism. These substances enhance the permeability of a capillary vessel of the external ear and thereby bring about inflammation.

As a result of enhancement of the permeability of the vessel, the pigment leaks out from the external ear. The amount of leaking pigment is proportional to the strength of inflammatory reaction. Therefore, the strength of inflammatory reaction can be determined by extracting the pigment from the ear and then measuring its concentration.

Results of the animal experiments are as follows.

Experiment 1

The effect of iodine-enriched egg on inflammation caused by arachidonic acid was examined with using 60 mice of ddY descent, which were divided into 6 groups each of 10 mice.

The right external ear of each mouse of each group was coated with 2 mg of arachidonic acid under light ether anesthesia. Then, a physiological saline solution containing 1 % of Evans blue was given through a tail vein in the amount of 0.1 ml per 10g bodyweight. Evans blue pigment leaked out on the external ear with the caused inflammation, and its leakage was measured.

In quantifying the leaked Evans blue pigment, 1 ml of 1.2 N-KOH was added to the external ear, and then hydrolysis was carried out at 37°C for 24 hours. Then, 4.5 ml of 5 : 13 acetone/phosphoric acid solution was added to the product of hydrolysis for removing protein and extracting the pigment. The quantity of pigment leaked on the external ear was measured by using a spectrophotometer at 620 nm.

The mice in each group were fed with commercially available solid feed and water under free or voluntary ingestion. The mice in the first group as a contrast group (AA Group) were treated by arachidonic acid. The mice in the second group as a control group (AA + OE Group) were treated by arachidonic acid and fed with ordinary egg. The mice in the third group as a test group (AA + IEE Group) were treated by arachidonic acid and fed with iodine-enriched egg.

The mice in the fourth group as a control group (AA + NDGA Group) were treated by arachidonic acid and dosed with nordihydroguaiaretic acid (NDGA). The mice in the fifth group as a control (AA + AS Group) were treated by arachidonic acid and dosed with aspirin. The mice in the sixth group as a contrast group (Non-Treated Group) were never treated by arachidonic acid.

The mice in group (AA + IEE) were given orally 0.3 g of iodine-enriched egg powder, which was obtained in the manner of Example 3 and suspended in 0.1% carboxymethylcellulose (CMC), for 10 successive days. The mice in group (AA + OE) were given 0.3 g of ordinary egg powder in the same manner as the iodine-enriched egg powder.

NDGA is an impedient or suppressive substance against 5-lipoxygenase as a metabolism enzyme to leucotriene (LT) of metabolism product from arachdonic acid. Aspirin is an impedient or suppressive substance against cycloxygenase as metabolism enzyme to prostaglandin (PC) of metabolism product from arachidonic acid.

NDGA was dissolved in deionized water, and was dosed to the mice in the group (AA + NDGA) by coating on the earlobe in the amount of 2 mg per mouse one hour before coating of arachidonic acid. Aspirin was suspended in 1 % carboxymethylcellulose (CMC), and orally dosed to the mice in the group (AA + AS) one hour before coating of arachidonic acid.

Table 1 shows results of the animal experiment. It is confirmed that the composite of the invention could significantly suppress the inflammation caused by arachidonic acid, like as NDGA (5-lipoxygenase impeder).

Table 1

| Effect on inflammation caused by arachidonic acid | | |
|---|---|---|
| Group | External ear weight (g) | Pigment leakage (μg/ear) |
| AA | 0.28 ± 0.04 | 65 ± 20 |
| AA + OE | 0.25 ± 0.04 | 50 ± 18 |
| AA + IEE | 0.22 ± 0.02* | 45 ± 10* |
| AA + NDGA | 0.19 ± 0.03** | 30 ± 10** |
| AA + AS | 0.30 ± 0.05 | 30 ± 20 |
| Non-Treated | 0.12 ± 0.01** | 8 ± 1** |

\* $p < 0.05$ and
\*\* $p < 0.01$ as compared with AA group.

Experiment 2

It is well known in the art that adrenal hormone is related to arachidonic acid metabolism. Accordingly, effects of iodine-enriched egg on adrenal body were examined.

The examination was conducted by using 30 mice of ddY descent, which were divided into 3 groups each of 10 mice. The mice in the first group as a contrast (AA Group) were treated by arachidonic acid. The mice in the second group as a test group (AA + IEE Group) were treated by arachidonic acid and fed with iodine-enriched egg. The mice in the third group as a control group (AA + DM Group) were treated by arachidonic acid and dosed with dexamethasone as an adrenal steroid hormone agent.

Arachidonic acid treatment to each mouse in each group was done by coating it on the right external ear in the amount of 2 mg under light ether anesthesia, like as in Experiment 1. The mice in each group were fed with commercially available solid feed under free or voluntary ingestion. The mice in the group (AA + IEE) were given orally 0.3 g of iodine-enriched egg powder, which was obtained by the manner of Example 3 and suspended in 0.1% carboxymethylcellulose (CMC) , for 10 successive days. The mice in the group (AA + DM) were dosed with dexamethasone (1 mg/kg B.W.) for 10 successive days.

Thereafter, the quantity of corticosterone was measured by a high performance liquid chromatography (HPLC). Table 2 shows the results of measurement. As shown, the composite according to the invention has anti-inflammatory property and is useful in significantly increasing corticostrone in blood without suppressing the adrenal function.

Table 2

| Changes in secretion of adrenal steroid hormone against inflammation caused by arachidonic acid | |
| --- | --- |
| Group | Corticosterone content (ng/ml serum) |
| AA | 195 ± 50 |
| AA + IEE | 320 ± 75** |
| AA + DM | 40 ± 30* |

* $p < 0.05$ and
** $p < 0.01$ as compared with AA group.


Experiment 3

Allergic asthma rats were produced by Murayama's method, in which rats were sensitized by absorbing $\alpha$-amylase derived from hay bacillus for 7 successive days. The rats were divided into 3 groups.

The rats in the first group as a contrast group (Non-Exposed + Non-IEE Group) were never exposed to an inflammatory antigen and fed with the feed not containing any iodine compound. The rats in the second group as a control group (Exposed + Non-IEE Group) were exposed to the antigen and fed with the feed not containing any iodine compound. The rats in the third group as a test group (Exposed + IEE Group) were exposed to the antigen and dosed with iodine-enriched egg powder, which was obtained in the manner of Example 1 described later, in the amount corresponding to 2.5 times of iodine requirement for a rat by free or voluntary ingestion for 14 days.

Judgement on the challenging test by antigen absorption was conducted with rat's respiration curve, visual judgement (classified into 0 to IV degrees in accordance with the state of inflammation), and measurement of inflammation continuing time. The histamine amount in overall blood and the histamine amount in lung were measured by Pruzansky-Patterson's method.

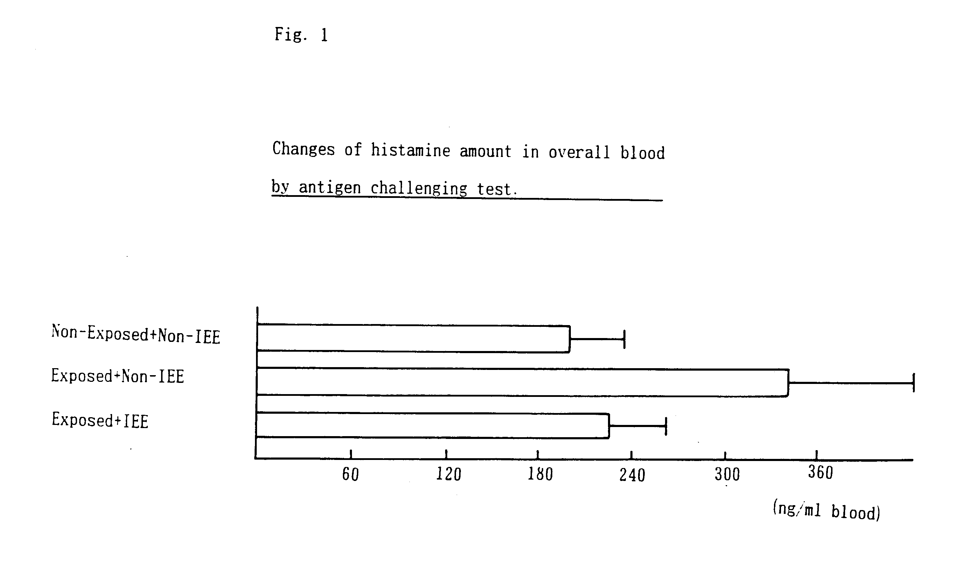

According to the general and overall judgement on challenging test, it was recognized that rat's asthma attach was notably suppressed in the group (Exposed + IEE) in comparison to the group (Exposed + Non-IEE). As shown in Figure 1, with respect to the histamine amount in overall blood, the amount in the group (Non-Exposed + Non-IEE) was lower than the amount in the group (Exposed + IEE), and the amount in the group (Exposed + IEE) was lower than the amount in the group (Exposed + Non-IEE).

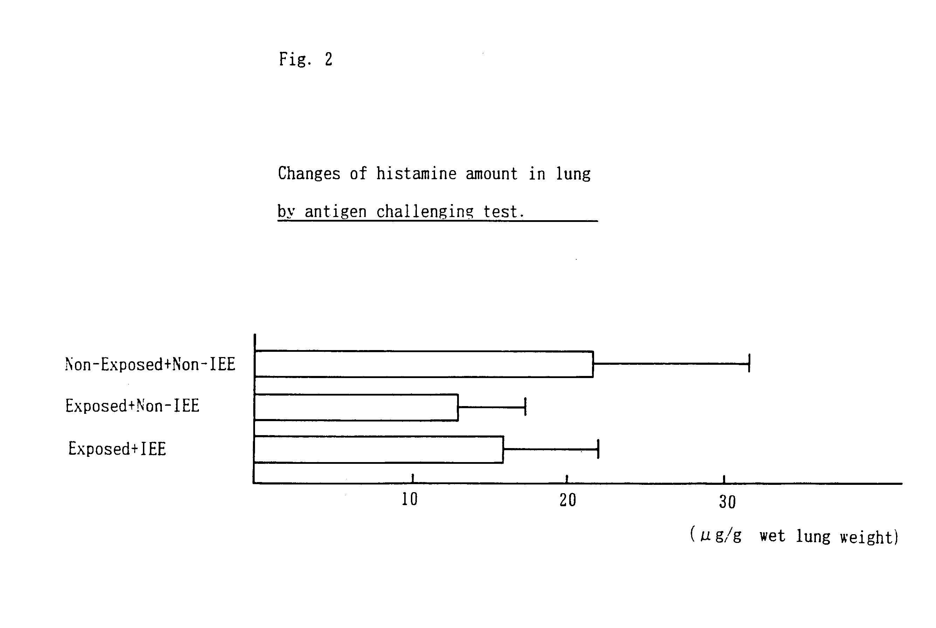

As shown in Figure 2, with respect to the histamine amount in lung, the amount in the group (Non-Exposed + Non-IEE) was higher than the amount in the group (Exposed + IEE), and the amount in the group (Exposed + IEE) was higher than the amount in the group (Exposed + Non-IEE).

The above results show that the composite of the invention is effective in suppressing allergic asthma attack in rats, which is confirmed by the free or isolated histamine amount.

Experiment 4

In order to prepare allergic rhinitis rats, group of 5 to 10 male Wistar rats each (weight range: 200 to 250g) were sensitized with an eightfold dilution of anti-bovine serum albumin rat serum (anti-BSA serum with an anti-body titer of x64) which was injected through the lateral tail vein. At 24 hours after injection, the passively sensitized rat was trachetomized under pentobarbital anesthesia and, after securing the airway, a polyethylene tube was passed through the trachea into the nasal cavity. Upon ligation of the esophagusand the trachea rostral to the fenestration, the nasal cavity was perfused with physiological saline of 37 °C at a flow rate of 0.25 ml/min. by means of a peristaltic pump. Perfusates draining from the nasal cavity were collected in consecutive 10-minute fractions. The solution used for perfusion varied serially in composition as follows (designated as P-1 to P-6):

Period 1 (P-1): Physiological saline for 10 minutes for nasal cavity washing and stabilization;

Period 2 (P-2): Physiological saline for 10 minutes to collect spontaneously draining perfusate after a 0.5-ml/100g b.w. intravenous dose of saline containing 4% pontamine sky blue (PSB), to determine PSB in the perfusate;

Period 3 (P-3): physiological saline containing 10 mg BSA per ml for 10 minutes, to provoke allergic reaction;

Period 4 (P-4): Physiological saline for 10 minutes;

Period 5 (P-5): Physiological saline for 10 minutes;

Period 6 (P-6): Physiological saline for 10 minutes. The amount of PSB leaked in the perfusate collected was determined spectrophotometrically at 620 nm.

The rats were devided into four experimental groups as follows:

(1) passively sensitized but no other treatment prior perfusion experiment (Challenge group);

(2) dosed orally with IE-egg (iodine-enriched egg obtained by the same method as Example 2) yolk suspension in 0.5% carboxymethylcellulose sodium solution in water at a dosage level of 153 $\mu$g/rat/day (corresponding to 100 times the daily iodine requirement for rats) once daily for 5 days ,followed by passive sensitization and subsequent perfusion experiment (IEE 5-Days Group);

(3) dosed orally with IE-egg (iodine-enriched egg obtained by the same method as Example 2) yolk suspension in 0.5% carboxymethylcellulose sodium solution in water at a dosage level of 153 $\mu$g/rat/day (corresponding to 100 times the daily iodine requirement for rats) once daily for 10 days, followed by passive sensitization and subsequent perfusion experiment (IEE 10-Days Group); and

(4) passively sensitized, and injected intraperitoneally with 25mg/kg of cepharanthine, a positive control, at 20 minutes prior to anesthesia for tracheotomy (CR group).

Lysosomal enzyme activity in perfusate was determined. Namely, nasal cavity perfusates were assayed for leaked lysosomal $\beta$-Glucuronidase activity by means of Fluorometrical method of Griffiths et al.

Rat nasal perfusate PSB data and lysosomal $\beta$-Glucuronidase assay data were statistically analyzed for evaluation of efficacy by using Student's t-Test.

Transudation content of pontamine sky blue (PSB) after antigen perfusion was significantly supressed throughout stage P-3 to P-6 both in IEE 5-Days Group and IEE 10-Days Group, comparing with Challenge Group, as shown in Table 3. The leakage content of PSB of the positive control, namely CR Group, was also significantly suppressed.

Evaluating $\beta$-Glucuronidase activity in the perfusate after antigen challenge, perfusion, there was no significant difference in all the groups at the stage P-3 in comparison with the stage P-2, but the activity did not increase in IEE 5-Days Group, IEE 10-Days Group and the CR Group, as shown in Table 4.

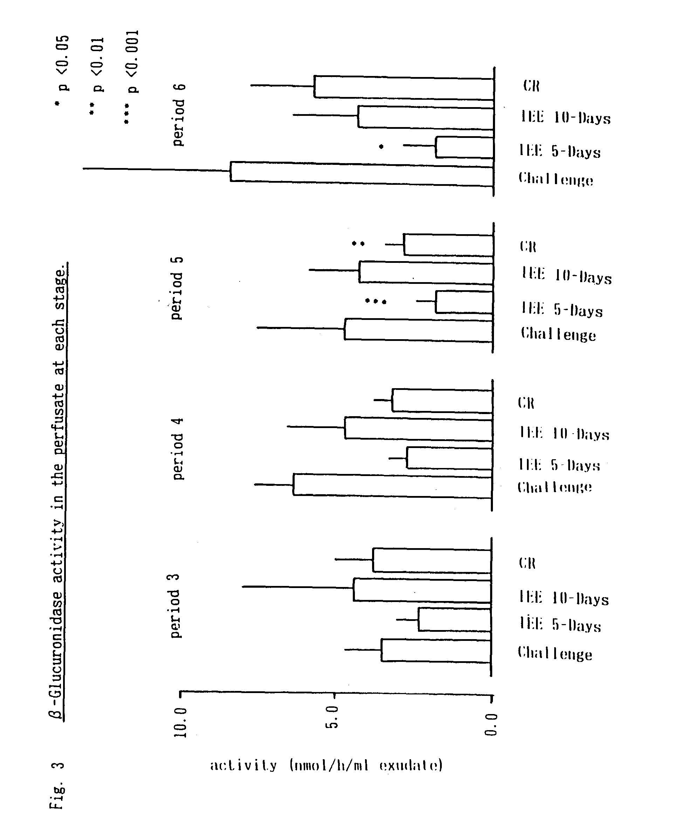

$\beta$-Glucuronidase activity of IEE 5-Days Group was significantly decreased at stage P-4 to P-6, as compared with the Challenge Group, as shown in Fig. 3. In the CR Group anti IEE 10-Days Group, $\beta$-Glucuronidase activity generally tended to be decreased, partly significantly decreased.

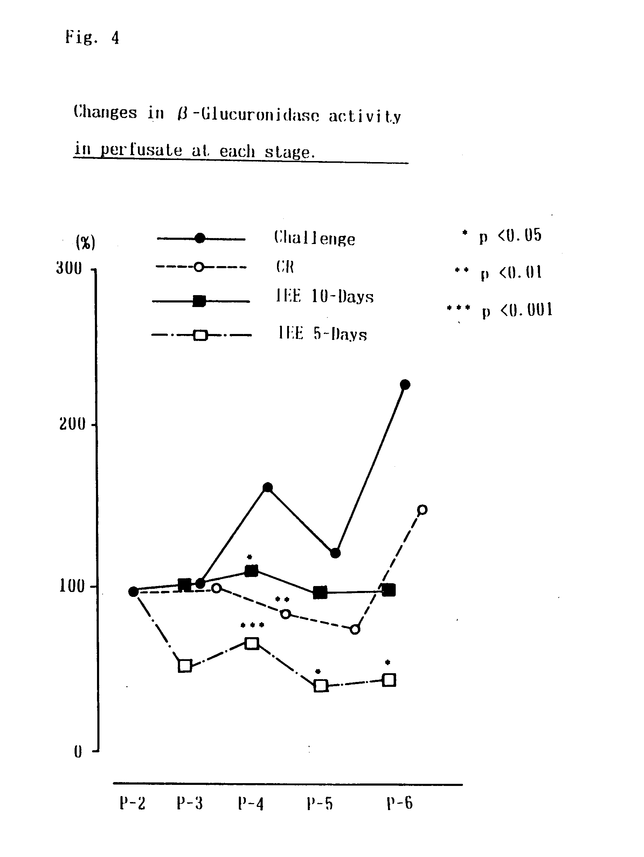

Changes in $\beta$-Glucuronidase activity in perfusate at each stage, as compared with stage P-2, are shown in Fig. 4.

After all, there are obtained two findings; one is that dye transudation from the nasal cavity, which would occur in sensitized rats a result of reaction between an antigen and an antibody, was suppressed by dosing iodine-enriched egg; and the other is that increase of $\beta$-Glucuronidase activity in the perfusate was also suppressed. These findings indicate that the iodine-enriched egg of the present invention can suppress and prevent allergic rhinitis of rats.

EP 0 472 003 A1

Table 3

Contents of pontamine sky blue in the exudate

in the perfusate after antigen challenge

| Group | Challenge | IEE 5-Days | IEE 10-Days | CR |
|---|---|---|---|---|
| P-3 | 0.734[a] ±0.497 | 0.123* ±0.183 | 0.559 ±0.289 | 0.498 ±0.255 |
| P-4 | 1.674 ±0.609 | 0.200*** ±0.275 | 0.879 ±0.684 | 0.550* ±0.389 |
| P-5 | 2.502 ±0.867 | 0.283* ±0.512 | 1.000** ±0.588 | 0.383** ±0.255 |
| P-6 | 3.520 ±1.229 | 0.567*** ±0.857 | 2.004* ±1.228 | 0.875* ±1.117 |

*$P<0.05$, **$P<0.01$, and ***$P<0.001$, as compared with Challenge in the same period.

[a] PSB $\mu$g/ml exudate, mean ± S.E.

7

Table 4

β-Glucuronidase activity in the perfusate

after antigen challenge

| Group | P-2 | P-3 | P-4 | P-5 | P-6 |
|---|---|---|---|---|---|
| Challenge | 3.80[a] ±0.70 | 3.53 ±1.29 | 6.31*** ±1.20 | 4.70 ±2.80 | 8.24 ±4.70 |
| IEE 5-Days | 4.32 ±1.55 | 2.31** ±0.74 | 2.71* ±0.65 | 1.78** ±0.64 | 1.83** ±1.02 |
| IEE 10-Days | 4.25 ±1.22 | 4.36 ±3.49 | 4.68 ±1.84 | 4.20 ±1.58 | 4.34 ±2.14 |
| CR | 3.96 ±1.48 | 3.76 ±1.27 | 3.15 ±0.59 | 2.86 ±0.52 | 5.74 ±1.96 |

*P<0.05, **P<0.01, and ***P<0.001, as compared with P-2 in the same

group.

[a] β-Glucuronidase activity nmol/h/ml exudate, mean ±S.D.

Clinical Test

Clinical test was done on seven subjects of outpatients with perennial allergic rhinitis,one male and six female. They were treated at the Department of Otorhinolaryngology, Tokyo Womens Medical college Second Hospital, Japan.

Their pharmacotherapeutic history is as follows; all patients had been receiving drugs year-round for therapeutic trials; the drugs given included antihistaminics, inhibitors of chemical mediator release, and occasionally short-term corticosteroid therapy. All the patients were taken off the said drugs during iodine-enriched egg diet period.

Each subject ingested a whole iodine-enriched egg (IEE), which was obtained by the same method as Example 1, daily as a dietary supplement during the period from March 3 to June 6. The iodine-enriched eggs were cooked in any desired way to patients' taste or uncooked.

Therapeutic efficacy of iodine-enriched egg was evaluated by parametrically scoring patients' responses to the questionnaire on the degree of subjective symptoms in accordance with the classification method of Hakamada and Nagai. Of the 7 patients, 6 patients became improved with alleviation in subjective symptoms by ingestion of iodine-enriched egg for 3 months. The effect of ingestion of iodine-enriched egg on nasal obstruction was evaluated in accordance with the method reported by Okuda et al.

Lysosomal enzyme activity in nasal discharge or pituita was collected from each patient in a Kumazawa rhinorrheal collection bottle before ingestion and after completion of iodine-enriched egg diet period of three months. Then, the specimens were assayed for lysosomal $\beta$-Glucuronidase activity by means of the Fluorometrical method of Griffiths et al. The lysosomal $\beta$-Glucuronidase assay data was statistically analyzed as to efficacy by using Student's t-Test.

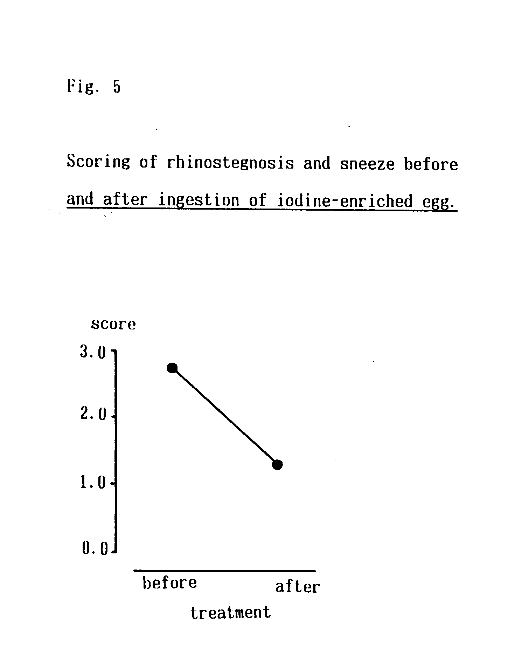

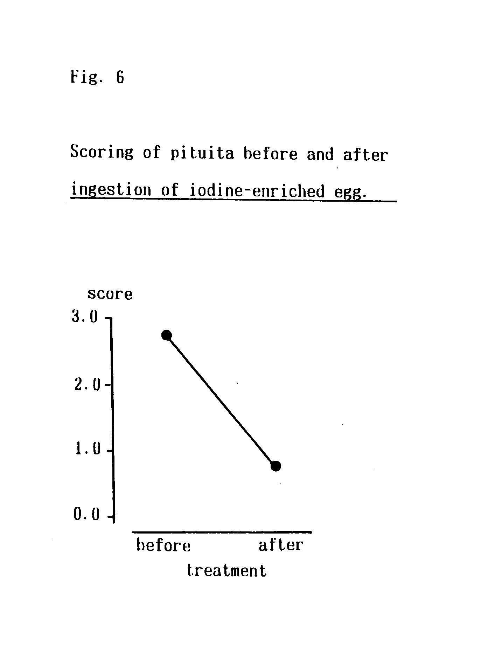

Efficacy of iodine enriched-egg ingestion was evaluated as to sneezing and rhinorrhea or pituita, by using parametric scoring in accordance with the report of Hakamada and Nagai. As shown in Fig. 5, the result is that a mean score after ingestion of iodine-enriched egg was 1.29, while a mean score before ingestion thereof was 2.71; thus an improvement rate was 52.7%. With respect to efficacy on nasal obstruction or pituita, a mean score before ingestion of iodine-enriched egg was 2.71, while a mean score after ingestion thereof was 0.71, as shown in Fig. 6. Thus an improvement rate was 73.7% . Consequently, an improvement rate of allergic rhinitis by ingestion of iodine-enriched egg was evaluated as a class of "effective" according to the report of Hakamada and Nagai.

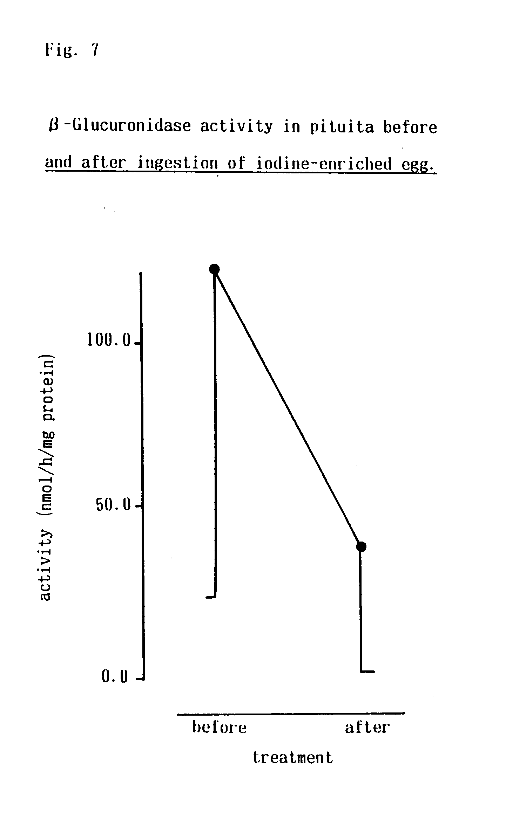

$\beta$-Glucuronidase activity in pituita of patients tended to decline by ingesting of iodine-enriched egg. Namely, the activity value after ingestion of iodine-enriched egg was 39.6 ±36.5 nmol/h/mg protein, while the activity value before ingestion thereof was 122.3 ±97.3 nmol/h/mg protein, as shown in Fig. 7. These findings indicate that iodine-enriched egg is effective in treatment of patients with allergic rhinitis.

Example 1

Potassium iodate was added to a commercially available feed for hens so that the iodine content became 20 ppm, and the feed was fed to hens which had begun to lay eggs three months ago. Thus six days after the feeding, iodine-enriched eggs were obtained. The iodine-enriched eggs contained iodine of 100 $\mu$g per egg on an average.

Example 2

Potassium iodate was added to a commercially available hen feed so that the iodine content became 40 ppm, and the feed was fed to hens which had begun to lay eggs three months ago. Thus six days after the feeding, iodine-enriched eggs were produced. The iodine-enriched egg contained iodine of 300 $\mu$g per egg on an average.

Example 3

Calcium iodate was added to a commercially available hen feed so that the iodine content became 1000 ppm, and powdered seaweed was further added to the admixture by 1%. The feed was fed to hens of 200 heads which had begun to lay eggs four months ago. The eggs of 200 kg obtained by the said feeding were dried by a spray-dryer, and thereby the composite of 45 kg of the invention was produced. The composite in the form of powdered egg contained iodine of 265 mg/kg.

**Claims**

1. Use of an iodine-enriched egg in the manufacture of a medicine for the treatment of inflammatory diseases.

2. Use of an iodine-enriched egg containing at least about 100 $\mu$g of iodine per egg in the manufacture of a medicine for the treatment of inflammatory diseases.

3. Use of a fowl's egg or eggs laid with an enriched iodine content of at least 100 $\mu$g per egg for the manufacture of a medicament for the treatment or prevention of inflammatory diseases.

Fig. 1

Changes of histamine amount in overall blood

by antigen challenging test.

Non-Exposed+Non-IEE

Exposed+Non-IEE

Exposed+IEE

60    120    180    240    300    360

(ng/ml blood)

10

Fig. 2

Changes of histamine amount in lung

by antigen challenging test.

Non-Exposed+Non-IEE

Exposed+Non-IEE

Exposed+IEE

10    20    30

( μg/g  wet lung weight)

Fig. 3    β-Glucuronidase activity in the perfusate at each stage.

Fig. 4

Changes in β-Glucuronidase activity
in perfusate at each stage.

Fig. 5

Scoring of rhinostegnosis and sneeze before
and after ingestion of iodine-enriched egg.

Fig. 6

Scoring of pituita before and after
ingestion of iodine-enriched egg.

Fig. 7

β-Glucuronidase activity in pituita before
and after ingestion of iodine-enriched egg.

European
Patent Office

EUROPEAN SEARCH
REPORT

Application Number

EP 91 11 2584

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOSIS PREVIEWS DATABASE BIOSIS, Philadelphia, PA. KOHNO H. et al:"Effect on iodine enriched-egg on nasal allergy basic and clinical investigations" Astract Nb: 83017344 * The whole document & FOLIA PHARMACOL.JPN 88 (3), 1986, p 223-228 * | 1-3 | A 61 K 35/54 A 61 K 33/18 |
| X | CH-A-621 243 (NIHON NOUSAN KOUGYOU) * page 2, lines 60 - 65 * * page 3, lines 5 - 12 * | 1-3 | |
| A | FR-M-3 979 (MASQUELIER J.) * page 2, column 1, lines 6 - 7 * | 1-3 | |
| A | EP-A-0 035 882 (NIHON NOSAN KOGYO) * page 1, lines 7 - 8 * | 1-3 | |
| A | DE-A-1 692 489 (SIEVERDING L.) * page 1, lines 8 - 11 * | 1-3 | |
| A | DE-A-3 040 780 (NIHON NOUSAN KOUGYOU) * abstract * | 1-3 | |
| A | DE-A-3 421 449 (NIHON NOSAN KOGYO) * abstract * | 1-3 | TECHNICAL FIELDS SEARCHED (Int. Cl.5) |
| A | US-A-4 394 376 (HIROSHI K. ET AL) * abstract * | 1-3 | A 61 K |
| A | US-A-4 485 098 (KUNITOSHI S. ET AL) * abstract * | 1-3 | |
| A | FR-M-857 (COUVERT J. ET AL) * page 1, column 1, lines 7 - 11 * * page 1, column 2, lines 5 - 8 * | 1-3 | |

The present search report has been drawn up for all claims

| Place of search | Date of completion of search | Examiner |
|---|---|---|
| Berlin | 07 November 91 | AVEDIKIAN P.F. |